# EUROPEAN PATENT APPLICATION

(11) **EP 2 006 283 A2**
(43) Date of publication of application: **24.12.2008**
(21) Application number: 07714971.4
(22) Date of filing: 27.02.2007
(51) Int. Cl.: C07D 201/04, C07D 231/10, C07D 223/10, C07D 255/02, C07B 61/00

(54) **PROCESS FOR PRODUCTION OF AMIDES OR LACTAMS**

(30) Priority: 10.03.2006 JP 2006065297; 18.03.2006 JP 2006075754; 08.09.2006 JP 2006244862
(71) Applicant: Mainichi Intecio., Osaka-shi, Osaka 530-0001 (JP)
(72) Inventor: ISHII, Yasutaka, Takatsuki-shi Osaka, 5691112 (JP); NAKANO, Tatsuya, Himeji-shi, Hyogo, 6711283 (JP); IWAHAMA, Takahiro, Himeji-shi, Hyogo, 6711283 (JP)
(74) Representative: Grünecker, Kinkeldey, Stockmair & Schwanhäusser Anwaltssozietät
(86) International application number: PCT/JP2007/053578
(87) International publication number: WO 2007/105482

(57) **Abstract**

An amide or lactam is produced by conducting a rearrangement of a corresponding oxime compound in the presence of a cyclic compound containing a structure represented by following Formula (1) as a ring constituent and a fluorinated alcohol: 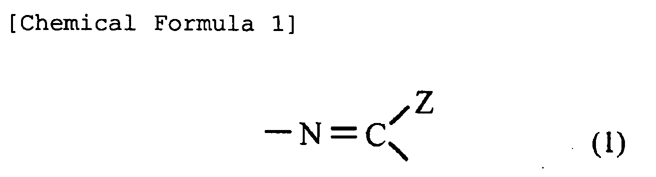 wherein Z represents a halogen atom or an -OR group, where R represents an organic group.
Z is preferably chlorine atom. Exemplary fluorinated alcohols include fluorine-containing branched-chain aliphatic alcohols represented by following Formula (3): 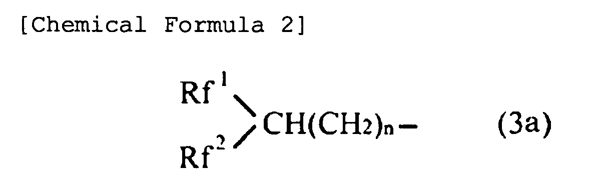 wherein Rf¹ and Rf² may be the same as or different from each other and each represent a perfluoroalkyl group having one to eight carbon atoms; and "n" denotes an integer of 0 to 8.

According to this process, amides or lactams can be simply produced in high yields without causing large amounts of by-products.

## Description

### Technical Field

The present invention relates to processes for the production of lactams or amides that are useful typically as raw materials for pharmaceutical drugs, agricultural chemicals, dyestuffs, polyamides and the like, or as solvents. More specifically, it relates to processes for producing the amides or lactams through rearrangement reactions of oxime compounds.

### Background Art

Techniques for producing amides or lactams from corresponding oxime compounds as raw materials through "Beckmann rearrangement" are industrially very important. The production of these compounds has been conducted using a process of acting fuming sulfuric acid in a stoichiometric amount or more, and this process raises an issue of byproduct of large amounts of ammonium sulfate to be treated.

To avoid this problem, there has been proposed the use of an aromatic compound, such as 2,4,6-trichloro-1,3,5-triazine, having a halogeno group that acts as a leaving group and two or more electron-withdrawing groups, as a Beckmann rearrangement catalyst. The catalyst applies less load on the environment (Non-patent Document 1). According to this technique, however, products are not easily separated and purified, because the technique requires use of a large amount of the catalyst or addition of an inorganic salt such as zinc chloride as a cocatalyst, for smoothly proceeding the reaction and yielding a target amide or lactam in a high yield.

[Non-patent Document 1] J. AM. CHEM. SOC. 2005, 127, 11240-11241

### Disclosure of the Invention

### Problems to be Solved by the Invention

An object of the present invention is to provide a process that allows a rearrangement reaction of an oxime compound with no generation of large amounts of by-products such as ammonium sulfate to thereby produce an amide or lactam in a high yield and a simple method.
Another object of the present invention is to provide a process that smoothly allows a rearrangement reaction of an oxime compound with a catalyst and a cocatalyst, both of which will be easily removed after the completion of reaction, to thereby produce an amide or lactam with a high degree of purification in a simple method.

### Means for Solving the Problems

After intensive investigations to achieve the above objects, the present inventors have found that the objects are achieved by the combination of a cyclic compound, containing a specific structure as a ring constituent, with a fluorinated alcohol. The present invention has been made based on these findings.

Specifically, the present invention provides a process for the production of amides or lactams, which process includes the step of carrying out a rearrangement of an oxime compound in the presence of a fluorinated alcohol and a cyclic compound containing a structure represented by following Formula (1) as a ring constituent to yield a corresponding amide or lactam:

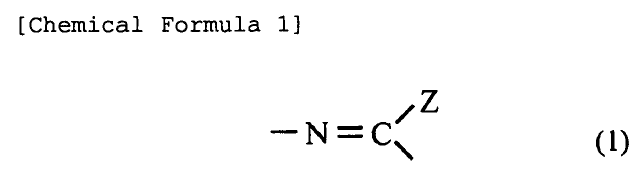

In Formula (1), Z represents a halogen atom or an -OR group, where R represents an organic group.

Z is preferably chlorine atom.

Exemplary preferred fluorinated alcohols include fluorine-containing branched-chain aliphatic alcohols represented by following Formula (3):

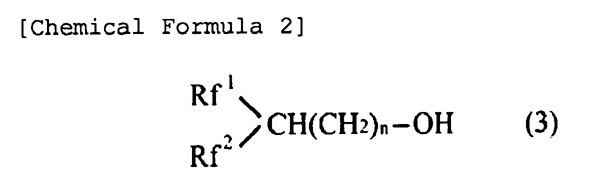

In Formula (3), Rf¹ and Rf² may be the same as or different from each other and each represent a perfluoroalkyl group having one to eight carbon atoms; and "n" denotes an integer of 0 to 8.

In the cyclic compound containing the structure represented by Formula (1) as a ring constituent, the organic group R is preferably a fluorine-containing branched-chain aliphatic group represented by following Formula (3a):

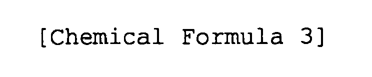

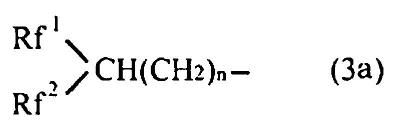

In Formula (3a), Rf¹ and Rf² may be the same as or different from each other and each represent a perfluoroalkyl group having one to eight carbon atoms; and "n" denotes an integer of 0 to 8.

Exemplary oxime compounds include oxime compounds each represented by following Formula (4) or (5):

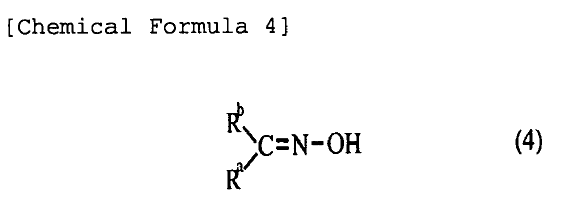

In Formula (4), R^{a} and R^{b} each independently represent an organic group, and one of R^{a} and R^{b} may be hydrogen atom,

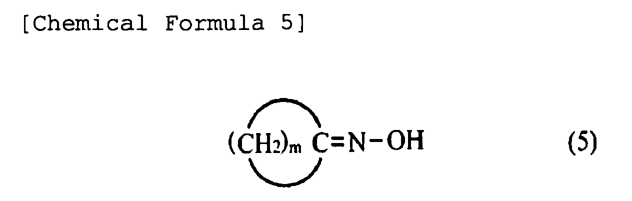

In Formula (5), "m" denotes an integer of 2 or more.

In the cyclic compound containing the structure represented by Formula (1) as a ring constituent, the organic group R is preferably a group corresponding to the oxime compound and represented by following Formula (4a) or (5a):

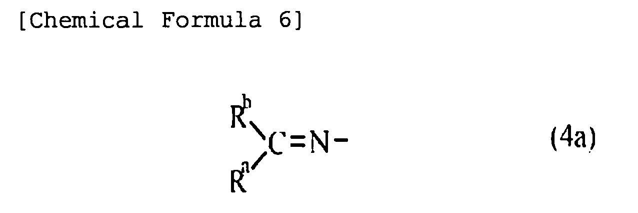

In Formula (4a), R^{a} and R^{b} each independently represent an organic group, and one of R^{a} and R^{b} may be hydrogen atom,

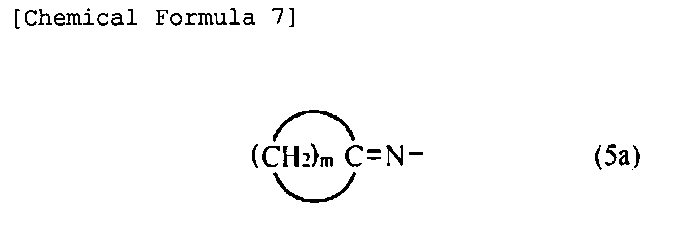

In Formula (5a), "m" denotes an integer of 2 or more. Advantages

According to the present invention, amides or lactams can be simply or easily produced in high yields while eliminating problems on removal or disposal of by-products generated in known processes for the production of amides or lactams, because rearrangement reactions of oximes can be carried out without generation of large amounts of by-products such as ammonium sulfate.
Use of a fluorinated alcohol as a cocatalyst helps to accelerate the reaction and to reduce the amount of catalysts. The fluorinated alcohol is easily separable from a product after the completion of the reaction and helps to save time and effort for purifying the product to thereby simply produce an amide or lactam with a high degree of purification.

### Best Mode for Carrying Out the Invention

According to the present invention, a cyclic compound containing a structure represented by following Formula (1) as a ring constituent is used as a catalyst, and a fluorinated alcohol is coexisted, so that a rearrangement reaction of an oxime compound rapidly and efficiently proceed to thereby give a corresponding amide or lactam in a high yield:

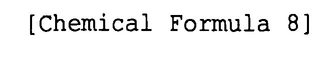

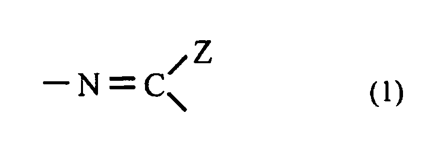

In Formula (1), Z represents a halogen atom or an OR group, where R represents an organic group.

Exemplary halogen atoms as Z in Formula (1) include fluorine atom, chlorine atom, bromine atom, and iodine atom, of which chlorine atom is preferred. The organic group as R in Formula (1) is not particularly limited, but it is preferably, for example, a group represented by following Formula (2), an alkyl group, or a haloalkyl group:

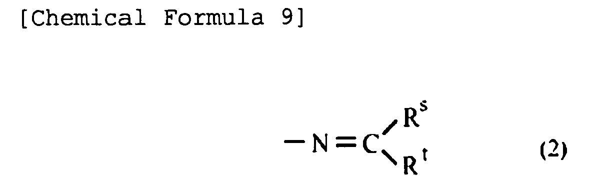

In Formula (2), R^{s} and R^{t} are the same as or different from each other and each represent a hydrocarbon group, and R^{s} and R^{t} may be combined to form a nonaromatic ring with the carbon atom to which R^{s} and R^{t} are bound.

Exemplary hydrocarbon groups as R^{s} and R^{t} include, but are not limited to, aliphatic chain groups, such as alkyl groups having about one to ten carbon atoms, alkenyl groups, and alkynyl groups; as well as cycloalkyl groups, aryl groups, and aralkyl groups. An example of the nonaromatic ring formed by R^{s}, R^{t} and the carbon atom to which R^{t} and R^{s} are bound includes a cycloalkyl group. In this case, the group represented by Formula (2) is a cycloalkylideneamino group. When the organic group R is a group represented by Formula (2), the group may be a group corresponding to the oxime compound used as a starting material. Namely, the group is represented by Chemical Formula in which -OH group is removed from the chemical formula of the corresponding oxime compound.

Exemplary alkyl groups as R include straight- or branched-chain alkyl groups having one to ten carbon atoms, such as methyl, ethyl, propyl, isopropyl, butyl, isobutyl, s-butyl, t-butyl, pentyl, and hexyl. Exemplary haloalkyl groups as R include groups corresponding to the alkyl groups, except with one or more halogen atoms, such as fluorine, chlorine, bromine, and iodine, being substituted. Preferred haloalkyl groups include fluorinated alkyl groups substituted with fluorine atom, of which fluorine-containing branched-chain aliphatic groups represented by Formula (3a) are more preferred. When the group represented by R is a fluorinated alkyl group, the fluorinated alkyl group is often a group corresponding to the fluorinated alcohol to be used:

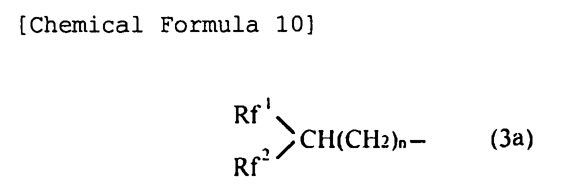

In Formula (3a), Rf¹ and Rf² may be the same as or different from each other and each represent a perfluoroalkyl group having about one to eight carbon atoms; and "n" denotes an integer of 0 to 8.

Exemplary usable cyclic compounds containing the structure represented by Formula (1) as a ring constituent include, but are not limited to, aromatic cyclic compounds or nonaromatic cyclic compounds each containing one or more of the structure per molecule. Among them, aromatic cyclic compounds are preferably used herein as cyclic compounds containing the structure represented by Formula (1) as a ring constituent. Exemplary aromatic cyclic compounds include triazine derivatives represented by following Formula (1a), pyrazine derivatives represented by following Formula (1b), pyrimidine derivatives represented by following Formula (1c), pyridazine derivatives represented by following Formula (1d), and pyridine derivatives represented by following Formula (1e):

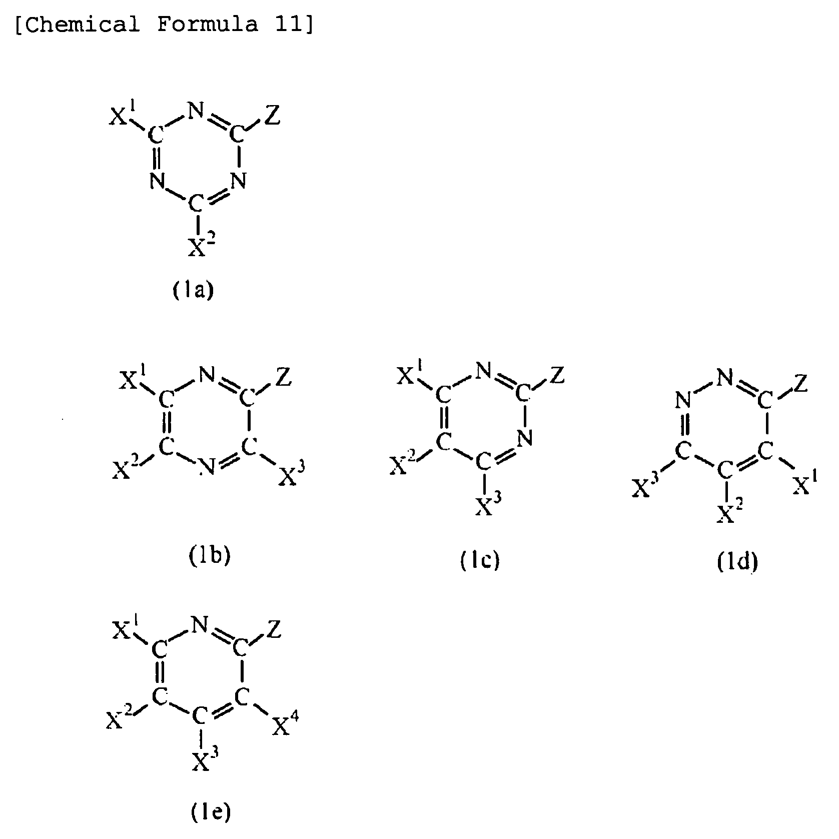

In Formulae (1a) to (1e), Z represents a halogen atom or an -OR group; R represents an organic group; X¹, X², X³, and X⁴ are the same as or different from one another and each represent hydrogen atom, a halogen atom, an alkyl group, a haloalkyl group (such as trifluoromethyl group, difluoromethyl group, or trichloromethyl group), an aryl group, a cycloalkyl group, hydroxyl group, an alkoxy group, an aryloxy group, a haloalkoxy group, mercapto group, carboxyl group, a substituted oxycarbonyl group, formyl group, an acyl group, an acyloxy group, nitro group, sulfo group, cyano group, amino group, oxyamino group, or another organic group, and at least two of X¹, X², X³, and X⁴ may be combined to form an aromatic or nonaromatic ring with atoms constituting the ring in the formulae.

Exemplary haloalkoxy groups as X¹, X², X³ and X⁴ include groups corresponding to fluorine-containing branched-chain aliphatic alcohols represented by Formula (3), such as hexafluoroisopropyloxy group (2,2,2-trifluoro-1-trifluoromethylethoxy group). The groups are represented by Chemical Formula in which hydrogen atom is removed from Formula (3) of the corresponding fluorine-containing branched-chain aliphatic alcohols. Exemplary haloalkoxy groups further include groups corresponding to fluorine-containing straight-chain aliphatic alcohols (fluorine-containing primary alcohols). The groups are represented by Chemical Formula in which hydrogen atom is removed from Formula (3) of the corresponding fluorine-containing straight-chain aliphatic alcohols. Exemplary other organic groups as X¹, X², X³ and X⁴ include groups corresponding to the groups represented by Formula (2), except with oxygen atom bound to the nitrogen atom thereof. X¹, X², X³ and X⁴ in the compounds represented by Formulae (1a) to (1e) may be the same groups as Z, namely, they may be groups selected from halogen atoms and -OR groups. Typically, triazine derivatives represented by Formula (1a), in which X¹ and X² are groups selected from halogen atoms and -OR groups, are cyclic compounds containing three of the structure represented by Formula (1) per molecule. Pyrazine derivatives represented by Formula (1b), pyrimidine derivatives represented by Formula (1c), and pyridazine derivatives represented by Formula (1d), in which X³ is a group selected from halogen atoms and -OR groups, are cyclic compounds containing two of the structure represented by Formula (1) per molecule.

More specifically, exemplary triazine derivatives represented by Formula (1a) include triazine derivatives having one or more halogen atoms (typically chlorine atoms) as substituents, such as 2-chloro-1,3,5-triazine, 2,4-dichloro-1,3,5-triazine, 2,4,6-trichloro-1,3,5-triazine (cyanuric chloride), 2-chloro-4,6-dihydroxy-1,3,5-triazine, 2-chloro-4,6-dinitro-1,3,5-triazine, 2-chloro-4-nitro-1,3,5-triazine, and 2-chloro-4,6-dioxymethyl-1,3,5-triazine; triazine derivatives having one or more haloalkoxy groups as substituents, such as 2-hexafluoroisopropyloxy-1,3,5-triazine, 2,4-bis(hexafluoroisopropyloxy)-1,3,5-triazine, and 2,4,6-tris(hexafluoroisopropyloxy)-1,3,5-triazine; triazine derivatives having one or more cycloalkylideneaminooxy groups as substituents, such as 2-cyclohexylideneaminooxy-1,3,5-triazine, 2-cyclododecylideneaminooxy-1,3,5-triazine, 2,4-bis(cyclohexylideneaminooxy)-1,3,5-triazine, 2,4-bis(cyclododecylideneaminooxy)-1,3,5-triazine, 2,4,6-tris(cyclohexylideneaminooxy)-1,3,5-triazine, and 2,4,6-tris(cyclododecylideneaminooxy)-1,3,5-triazine; triazine derivatives having at least one halogen atom and at least one haloalkoxy group as substituents, such as 2-chloro-4,6-bis(hexafluoroisopropyloxy)-1,3,5-triazine and 2,4-dichloro-6-(hexafluoroisopropyloxy)-1,3,5-triazine; triazine derivatives having at least one halogen atom and at least one cycloalkylideneaminooxy group as substituents, such as 2-chloro-4-cyclohexylideneaminooxy-1,3,5-triazine and 2-chloro-4-cyclododecylideneaminooxy-1,3,5-triazine; triazine derivatives having at least one cycloalkylideneaminooxy group and at least one haloalkoxy group as substituents, such as 2-cyclohexylideneaminooxy-4,6-bis(hexafluoroisopropyloxy)-1,3,5-triazine and 2-cyclododecylideneaminooxy-4,6-bis(hexafluoroisopropyloxy)-1,3,5-triazine; and triazine derivatives having at least one halogen atom, at least one haloalkoxy group, and at least one cycloalkylideneamino group as substituents, such as 2-chloro-4-(hexafluoroisopropyloxy)-6-cyclohexylideneaminooxy-1,3,5-triazine and 2-chloro-4-(hexafluoroisopropyloxy)-6-cyclododecylideneaminooxy-1,3,5-triazine.

Exemplary pyrazine derivatives represented by Formula (1b) include pyrazine derivatives having one or more halogen atoms as substituents, such as 2-chloropyrazine, 2,3-dichloropyrazine, and 2-chloro-3,5-dinitropyrazine; pyrazine derivatives having one or more haloalkoxy groups as substituents, such as 2-(hexafluoroisopropyloxy)pyrazine; and pyrazine derivatives having one or more cycloalkylideneaminooxy groups as substituents, such as 2-cyclododecylideneaminooxypyrazine.

Exemplary pyrimidine derivatives represented by Formula (1c) include pyrimidine derivatives having one or more halogen atoms as substituents, such as 2,4-dichloropyrimidine, 2,4,6-trichloropyrimidine, 4,6-dichloro-5-nitropyrimidine, and 2,4-dichloro-6-nitropyrimidine; pyrimidine derivatives having one or more haloalkoxy groups as substituents, such as 2,4-bis(hexafluoroisopropyloxy)pyrimidine; and pyrimidine derivatives having one or more cycloalkylideneaminooxy groups as substituents, such as 2,4-dicyclododecylideneaminooxypyrimidine.

Exemplary pyridazine derivatives represented by Formula (1d) include pyridazine derivatives having one or more halogen atoms as substituents, such as 3-chloropyridazine and 3,6-dichloropyridazine; pyridazine derivatives having one or more haloalkoxy groups as substituents, such as 3-hexafluoroisopropyloxypyridazine; and pyridazine derivatives having one or more cycloalkylideneaminooxy groups as substituents, such as 3-cyclododecylideneaminooxypyridazine.

Exemplary pyridine derivatives represented by Formula (1e) include pyridine derivatives having one or more halogen atoms as substituents, such as 2-chloro-3,5-dinitropyridine, 2,4,6-trichloropyridine, and 2-chloropyridine; pyridine derivatives having one or more haloalkoxy groups as substituents, such as 2-hexafluoroisopropyloxypyridine; and pyridine derivatives having one or more cycloalkylideneaminooxy groups as substituents, such as 2-cyclododecylideneaminooxypyridine.

Among them, triazine derivatives represented by Formula (1a) are preferred, of which 2,4,6-trichloro-1,3,5-triazine, 2,4,6-tris(hexafluoroisopropyloxy)-1,3,5-triazine, and 2,4,6-tris(cyclododecylideneaminooxy)-1,3,5-triazine are more preferred.

Exemplary cyclic compounds containing the structure represented by Formula (1) as a ring constituent further include compounds having a nitrogen-containing fused heterocyclic skeleton, such as quinoline, isoquinoline, quinazoline, quinoxaline, phthalazine, purine, pteridine, phenanthridine, and phenanthroline. Exemplary cyclic compounds further include aromatic or nonaromatic cyclic compounds containing at least one heteroatom other than nitrogen atom as a ring constituent and also containing the structure represented by Formula (1) as a ring constituent.

When the cyclic compound containing the structure represented by Formula (1) as a ring constituent is a compound having an -OR group as Z, the cyclic compound may be prepared beforehand and subjected to a reaction. In another embodiment, such a cyclic compound having an -OR group as Z is formed by incorporating a corresponding compound having a halogen atom as Z in combination with a compound that generates an RO⁻ ion into a reaction system for the production of an amide or lactam and thereby allowing a substitution reaction between the halogen atom and the -OR group to proceed in the reaction system. The compound that generates an RO⁻ ion is not particularly limited, but it is often a fluorinated alcohol used as a cocatalyst or an oxime compound used as a starting material herein. Specifically, exemplary embodiments of the present invention in which Z is an -OR group further include an embodiment in which a cyclic compound containing the structure represented by Formula (1) as a ring constituent, in which Z is a halogen atom, is allowed to react with a fluorinated alcohol to give a cyclic compound having a haloalkoxy group as a substituent in situ in the reaction system; and an embodiment in which a cyclic compound containing the structure represented by Formula (1) as a ring constituent, in which Z is a halogen atom, is allowed to react with an oxime compound to give a cyclic compound having, as a substituent, a group corresponding to the oxime compound and represented by Chemical Formula in which hydrogen atom is removed from the chemical formula of the corresponding oxime compound (e.g., a cycloalkylideneaminooxy group).

The amount of the compound containing the structure represented by Formula (1) as a ring constituent is, for example, about 0.0001 to 1 mole, preferably about 0.0005 to 0.5 mole, and more preferably about 0.001 to 0.2 mole, per 1 mole of oxime compounds. Each of different compounds containing the structure represented by Formula (1) as a ring constituent can be used alone or in combination.

Fluorinated alcohols for use herein are not particularly limited and include any aliphatic alcohols or aromatic alcohols with fluorine atom replacing part or all of hydrogen atoms of hydrocarbon moiety. Such fluorinated alcohols may be monohydric alcohols or polyhydric alcohols.

Exemplary fluorinated aliphatic alcohols include aliphatic chain alcohols and aliphatic cyclic alcohols. Exemplary preferred aliphatic chain alcohols include fluorine-containing straight-chain aliphatic alcohols as straight-chain alcohols having about one to twenty carbon atoms with fluorine atom replacing part or all of hydrogen atoms of hydrocarbon moiety; and fluorine-containing branched-chain aliphatic alcohols having about three to twenty carbon atoms with fluorine atom replacing part or all of hydrogen atoms of hydrocarbon moiety. In fluorinated aliphatic chain alcohols, their hydrocarbon moieties (hereinafter referred to as a "fluorinated hydrocarbon moieties") may contain one or more unsaturated bonds. Exemplary fluorine-containing straight-chain aliphatic alcohols with fluorine atom replacing part of hydrogen atoms of hydrocarbon moiety include 1,1-difluoroethanol, 1,1,2-trifluoroethanol, 2,2,2-trifluoroethanol, 1,1-difluoro-1-propanol, 1,2-difluoro-1-propanol, 1,2,3-trifluoro-1-propanol, 3,3,3-trifluoro-1-propanol, 1,1,2,2-tetrafluoro-1-propanol, 1,3-difluoro-1,3-propanediol, 2,3,4-trifluoro-1-butanol, 4,4,4-trifluoro-1-butanol, 3,3,4,4,4-pentafluoro-1-butanol, 1,1,2,2,3,3-hexafluoro-1-butanol, 1,1,2,2-tetrafluoro-1-butanol, 1,2,3,4-tetrafluoro-1-butanol, 3,3,4,4,4-pentafluoro-1-butanol, 1,2,3,4-tetrafluoro-1,4-butanediol, 1,1,2,2-tetrafluoro-1-pentanol, 5,5,5-trifluoro-1-pentanol, 4,4,5,5,5-pentafluoro-1-pentanol, 1,1,2,2-tetrafluoro-1-hexanol, and 5,5,6,6,6-pentafluoro-1-hexanol. Exemplary fluorine-containing aliphatic branched-chain alcohols include hexafluoroisopropanol, heptafluoroisopropanol, 3,3,3-trifluoro-2-trifluoromethyl-1-propanol, 2-trifluoromethyl-1-butanol, 2-trifluoromethyl-1,4-butanediol, and 2-trifluoromethyl-3,3,4,4,4-pentafluoro-1-butanol.

Exemplary fluorinated aliphatic cyclic alcohols usable herein include aliphatic cyclic alcohols having about three to twenty carbon atoms, such as cyclohexanol and cyclopentanol, with one or more fluorine atoms per molecule. The way to contain fluorine atoms is not particularly limited. Typically, fluorine atoms may be bound to a carbon atom constituting a ring; or a fluorine-containing hydrocarbon group may be bound to a carbon atom constituting a ring.

Exemplary usable fluorinated aromatic alcohols include aromatic alcohols, such as benzyl alcohol and phenylethanol, with one or more fluorine atoms per molecule. The way to contain fluorine atoms is not particularly limited. Typically, a fluorinated hydrocarbon group may be substituted on an aromatic ring, or a chain hydrocarbon moiety may have a fluorine atom.

Among these fluorinated alcohols, fluorine-containing branched-chain aliphatic alcohols represented by following Formula (3) are preferred, of which hexafluoroisopropanol is more preferred:

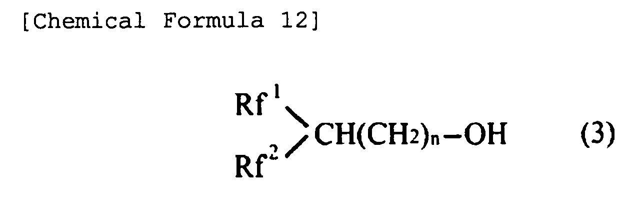

wherein Rf¹ and Rf² may be the same as or different from each other and each represent a perfluoroalkyl group having about one to eight carbon atoms; and "n" denotes an integer of 0 to 8.

In a preferred embodiment of the present invention, the fluorinated alcohol is a compound represented by Formula (3), and R in Formula (1) is a group represented by following Formula (3a):

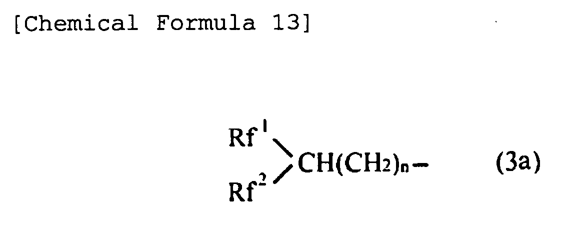

wherein Rf¹ and Rf² may be the same as or different from each other and each represent a perfluoroalkyl group having about one to eight carbon atoms; and "n" denotes an integer of 0 to 8.

The amount of fluorinated alcohols is not particularly limited and may be selected within a broad range of, for example, 0.001 mole or more, preferably 0.05 mole or more, and more preferably 0.5 mole or more, per 1 mole of oxime compounds. In an embodiment, fluorinated alcohols are used in large excess to oxime compounds. In another preferred embodiment, fluorinated alcohols are used as solvents. Each of different fluorinated alcohols may be used alone or in combination.

Oxime compounds for use herein as starting materials are not particularly limited and may be selected suitably according to an amide or lactam to be produced. Exemplary oxime compounds include compounds represented by following Formula (4) or Formula (5):

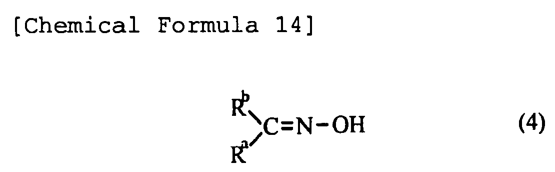

wherein R^{a} and R^{b} each independently represent an organic group, where one of R^{a} and R^{b} may be hydrogen atom,

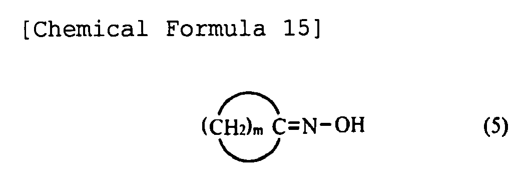

wherein "m" denotes an integer of 2 or more.

Organic groups as R^{a} and R^{b} include alkyl groups such as methyl, ethyl, propyl, isopropyl, butyl, isobutyl, s-butyl, t-butyl, pentyl, isopentyl, hexyl, isohexyl, heptyl, octyl, nonyl, decyl, dodecyl, and pentadecyl groups, of which those having one to twenty carbon atoms are preferred, those having one to twelve carbon atoms are more preferred, and those having two to eight carbon atoms are further preferred; alkenyl groups such as vinyl, allyl, 1-propenyl, 1-butenyl, 1-pentenyl, and 1-octenyl groups, of which those having two to twenty carbon atoms are preferred, those having two to twelve carbon atoms are more preferred, and those having about two to eight carbon atoms are further preferred; alkynyl groups such as ethynyl and 1-propynyl groups, of which those having two to twenty carbon atoms are preferred, those having two to twelve carbon atoms are more preferred, and those having about two to eight carbon atoms are further preferred; cycloalkyl groups such as cyclopropyl, cyclobutyl, cyclopentyl, cyclohexyl, cycloheptyl, cyclooctyl, and cyclododecyl groups, of which those having three to twenty carbon atoms are preferred, and those having three to fifteen carbon atoms are more preferred; cycloalkenyl groups such as cyclopentenyl, cyclohexenyl, and cyclooctenyl groups, of which those having three to twenty carbon atoms are preferred, and those having three to fifteen carbon atoms are more preferred; aryl groups such as phenyl and naphthyl groups; aralkyl groups such as benzyl, 2-phenylethyl, and 3-phenylpropyl groups; and aromatic or nonaromatic heterocyclic groups such as 2-pyridyl, 2-quinolyl, 2-furyl, 2-thienyl, and 4-piperidinyl groups. Each of these organic groups may have one or more substituents of every kind within ranges not adversely affecting the reaction. Exemplary substituents include halogen atoms, oxo group, hydroxyl group, mercapto group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, alkyl groups, alkenyl groups, alkynyl groups, cycloalkyl groups, cycloalkenyl groups, aryl groups (e.g., phenyl and naphthyl groups), aralkyl groups, and heterocyclic groups.

Exemplary oxime compounds represented by Formula (4) include acetaldehyde oxime, acetone oxime, 2-butanone oxime, 2-pentanone oxime, 3-pentanone oxime, 1-cyclohexyl-1-propanone oxime, benzaldehyde oxime, acetophenone oxime, benzophenone oxime, and 4'-hydroxyacetophenone oxime.

The ring in Formula (5) may have one or more substituents bound thereto, and/or may have one or more other rings fused thereto. In Formula (5), "m" is, for example, about 2 to 30, preferably about 4 to 20, and more preferably about 5 to 14. Exemplary cyclic oxime compounds represented by Formula (5) include cyclopropanone oxime, cyclobutanone oxime, cyclohexanone oxime, cycloheptanone oxime, cyclooctanone oxime, cyclononanone oxime, cyclodecanone oxime, cyclododecanone oxime, cyclotridecanone oxime, cyclotetradecanone oxime, cyclopentadecanone oxime, cyclohexadecanone oxime, cyclooctadecanone oxime, and cyclononadecanone oxime. Exemplary substituents which may combine with the ring contain the above-exemplified substituents which the above-mentioned organic groups may have.

In a preferred embodiment of the present invention, the oxime compound is represented by Formula (4), and group R in Formula (1) is represented by following Formula (4a). In another preferred embodiment, the oxime compound is represented by Formula (5), and group R in Formula (1) is represented by following Formula (5a):

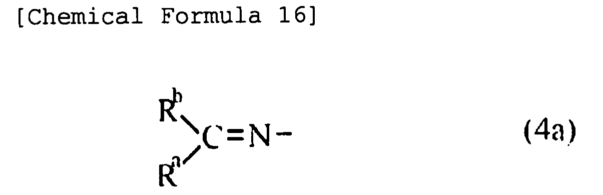

In Formula (4a), R^{a} and R^{b} each independently represent an organic group, where one of R^{a} and R^{b} may be hydrogen atom.

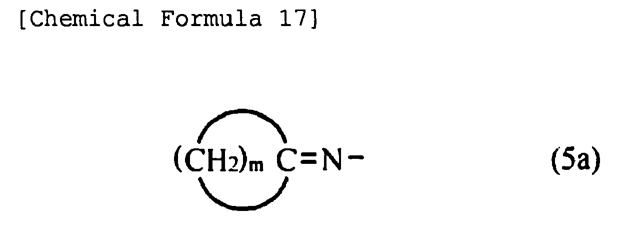

In Formula (5a), "m" denotes an integer of 2 or more.

Each of different oxime compounds may be selected and used alone or in combination.

Rearrangement reactions of oxime compounds are carried out in the presence of, or in the absence of solvents. The solvents may be the above-mentioned fluorinated alcohols or other solvents. The other solvents have only to be inert (inactive) under reaction conditions. Exemplary other solvents include organic acids such as acetic acid, propionic acid, and trifluoroacetic acid; nitriles such as acetonitrile, propionitrile, and benzonitrile; amides such as formamide, acetamide, dimethylformamide (DMF), and dimethylacetamide; aliphatic hydrocarbons such as hexane and octane; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents.

A reaction temperature is not particularly limited and may be set according typically to the types of oxime compounds to be used, and the types of catalysts and solvents. It is, for example, about 0°C to 250°C, preferably about 25°C to 150°C, and more preferably about 40°C to 120°C. A reaction may be carried out in an atmosphere of an inert gas such as nitrogen or argon gas, or can be carried out in an atmosphere of air or oxygen. Particularly, in a preferred embodiment of the present invention, the reaction is carried out in an air atmosphere under reflux conditions.

By using the process according to the present invention, an oxime compound represented by Formula (4) yields an amide compound represented by following Formula (6); and a cyclic oxime compound represented by Formula (5) yields a lactam represented by following Formula (7). More specifically, acetophenone oxime typically yields acetanilide; and a cycloalkanone oxime yields a lactam having one more number of members constituting a ring than the number of the members constituting a ring of original cycloalkanone oxime. Typically, cyclohexanone oxime yields ε-caprolactam; cycloheptanone oxime yields 7-heptanelactam; and cyclooctanone oxime yields 8-octanelactam:

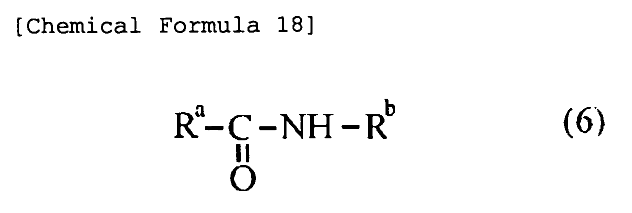

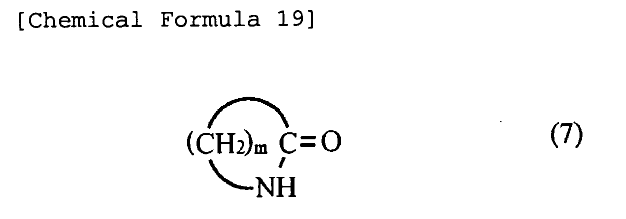

wherein "m" is as defined above.

Reaction products after the completion of reaction may be separated and purified by a separation procedure such as filtration, concentration, distillation, extraction, crystallization, recrystallization, adsorption, or column chromatography, or any combination of these separation procedures.

In a preferred embodiment, an oxime compound can be simply and efficiently prepared under mild conditions according to the following process. Further, the process is very advantageous because a reaction for the synthesis of the oxime compound and a reaction for the formation of an amide or lactam through rearrangement of the oxime compound can be carried out in one step without requiring an extra step of separating and purifying the oxime compound in the midway.

Specifically, an oxime compound is preferably prepared by reacting a compound having methyl group or methylene group with a nitrous ester or nitrite (salt of nitrous acid) in the presence of a nitrogen-containing cyclic compound containing, as a ring constituent, a skeleton represented by following Formula (8):

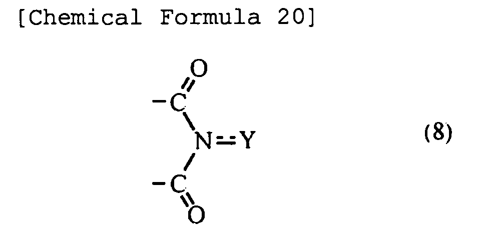

In Formula (8), Y represents an oxygen atom or an -OR' group, and R' represents hydrogen atom or a hydroxyl-protecting group.

Exemplary usable nitrogen-containing cyclic compounds containing the skeleton represented by Formula (8) as a ring constituent include N-hydroxyimide compounds derived from aliphatic polycarboxylic acid anhydrides (cyclic anhydrides) or aromatic polycarboxylic acid anhydrides (cyclic anhydrides), such as N-hydroxysuccinimide, N-hydroxyphthalimide, N,N'-dihydroxypyromellitic diimide, N-hydroxyglutarimide, N-hydroxy-1,8-naphthalenedicarboximide, N,N'-dihydroxy-1,8,4,5-naphthalenetetracarboxylic diimide; and compounds obtained by introducing a protecting group to the hydroxyl group of the corresponding N-hydroxyimide compounds.

Exemplary compounds having methyl group or methylene group include compounds represented by following Formula (9) :

[Chemical Formula 21]

R^{a}-CH₂-R^{b} (9)

wherein R^{a} and R^{b} are as defined above.

Specifically, exemplary compounds having methyl group or methylene group include ethane, propane, butane, pentane, hexane, heptane, octane, n-propylcyclohexane, toluene, p-xylene, ethylbenzene, isopropylbenzene, diphenylmethane, and 1,2-diphenylethane.

Exemplary compounds having methylene group further include compounds represented by following Formula (10):

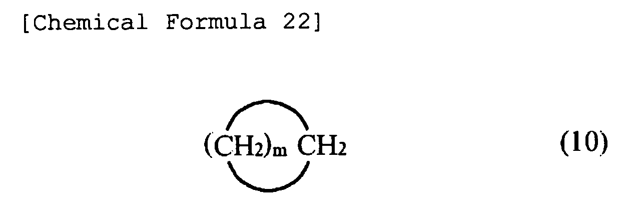

wherein "m" is as defined above.

The ring in Formula (10) may have one or more substituents thereon and/or may have one or more other rings fused thereto. Exemplary compounds represented by Formula (10) include cyclopropane, cyclobutane, cyclopentane, cyclohexane, cycloheptane, cyclooctane, cyclononane, cyclodecane, cyclododecane, cyclotridecane, cyclotetradecane, cyclopentadecane, cyclohexadecane, cyclooctadecane, and cyclononadecane. The substituents which may bond to the ring are as with the above-exemplified substituents which the organic groups may have.

The nitrous esters include alkyl nitrites such as methyl nitrite, ethyl nitrite, propyl nitrite, isopropyl nitrite, butyl nitrite, isobutyl nitrite, t-butyl nitrite, amyl nitrite, isoamyl nitrite, t-amyl nitrite, and hexyl nitrite; aryl nitrites such as phenyl nitrite; and aralkyl nitrites such as benzyl nitrite. Exemplary preferred nitrous esters include alkyl nitrites such as alkyl nitrites whose alkyl moiety has one to six carbon atoms. Exemplary nitrites (salts of nitrous acid) include ammonium nitrite; alkaline earth metal salts of nitrous acid, such as lithium nitrite, sodium nitrite, potassium nitrite, and barium nitrite; and other metal salts of nitrous acid, such as zinc nitrite.

The ratio of a compound having methyl group or methylene group to a nitrous ester or nitrite may be suitably set according typically to the types and combination of the two compounds. Typically, the compound having methyl group or methylene group may be used in an equivalent amount or in excess (e.g., about 1.1 to 50 times by equivalent weight or more, and preferably about 3 to 30 times by equivalent weight) to the nitrous ester or nitrite; or conversely, the nitrous ester or nitrite may be used in excess to the compound having methyl group or methylene group.

A reaction between a compound having methyl group or methylene group and a nitrous ester or nitrite is carried out in the presence of, or in the absence of, a solvent. The solvent herein is not particularly limited, and exemplary solvents include the above-exemplified solvents usable in rearrangement reactions of oxime compounds. A reaction temperature and other conditions are not particularly limited, and the reaction can be carried out under conditions as in the rearrangement reactions of oxime compounds. Typically, the reaction temperature is about 0°C to 250°C, preferably about 25°C to 150°C, and more preferably about 40°C to 120°C. The reaction may be carried out in an atmosphere of an inert gas such as nitrogen gas or argon gas. In some types of target products, the reaction may be carried out in an atmosphere of air or oxygen. The reaction can be carried out under reduced pressure, under normal pressure, or under a pressure (under a load), according to a common system such as batch system, semi-batch system, or continuous system (e.g., a multistage continuous circulation system). The reaction is preferably conducted under reduced pressure and is more preferably under such reduced pressure as to remove nitrogen oxide gases (typified by NO₂) by-produced in the reaction, e.g., at about 30 to 700 mmHg (3.99 to 93.1 kPa). This gives remarkably improved yields. This is probably because nitrogen oxide gases such as NO₂ adversely affect the reaction.

It is supposed that a reaction between a compound having methyl group or methylene group and a nitrous ester or nitrite initially gives a nitroso compound, and the nitroso compound undergoes rearrangement to give an oxime compound. Typically, it is supposed that a reaction between cyclohexane and a nitrous ester or nitrite initially gives nitrosocyclohexane, and this undergoes rearrangement to give cyclohexanone oxime. A nitroso compound of some type may be in equilibrium with a corresponding dimer (a di-N-oxide compound composed of two molecules of nitroso compound bonded through their nitrogen atoms), and the equilibrium may lie to the dimer. When the reaction is conducted for a long period of time, the nitroso compound and a dimer thereof can be in trace amounts and in yields of at most less than 1%.

In a preferred embodiment, a reaction between a compound having methyl group or methylene group and a nitrous ester or nitrite is conducted by adding the nitrous ester or nitrite sequentially (intermittently) or continuously to the reaction system. According to this technique, side reactions typically in nitrosation are suppressed to give a nitroso compound or a dimer thereof with high selectivity, as compared in the case where the nitrous ester or nitrite is added all at once. Accordingly, a subsequent rearrangement reaction typically gives an oxime compound in a high yield.

In another embodiment for forming an oxime compound in a high yield, reactions may be conducted stepwise by arranging a step of carrying out a reaction between a compound having methyl group or methylene group and a nitrous ester or nitrite to give a nitroso compound or a dimer thereof, and a step of converting the formed nitroso compound or a dimer thereof into the oxime compound. When this technique is employed, the total reaction period may be significantly reduced or shortened by adding additives to the reaction system or by carrying out heating in the latter conversion step (nitroso compound rearrangement step). The former nitrosation step and the latter rearrangement step may be conducted in different solvents. According to this technic, the latter nitrosation step is preferably conducted under reduced pressure to increase the yield remarkably, for the same reason as above.

The additives are not particularly limited, as long as they can induce a rearrangement from the nitroso form to the oxime form; and exemplary preferred additives include acids and bases. Exemplary acids include sulfonic acids such as methanesulfonic acid, trifluoromethanesulfonic acid, benzenesulfonic acid, and p-toluenesulfonic acid; mineral acids such as sulfuric acid, nitric acid, hydrochloric acid, phosphoric acid, boric acid, and fuming sulfuric acid; Lewis acids such as aluminum chloride, zinc chloride, and scandium triflate; solid acids such as silica, alumina, and zeolite; complex acids including polyacids such as phosphomolybdic acid, phosphotungstic acid, silicomolybdic acid, and silicotungstic acid; and strongly acidic cation-exchange resins. Exemplary bases include organic bases including tertiary amines such as triethylamine, nitrogen-containing heterocyclic compounds such as pyridine, as well as sodium acetate and sodium methoxide; inorganic bases such as sodium carbonate, sodium hydrogen carbonate, sodium hydroxide, and potassium hydroxide; and solid bases such as magnesium oxide, hydrotalcite, and hydroxyapatite. Such additives may be added all at once or in plural installments. The amount of additives is, for example, about 0.01 to 100 parts by weight, preferably about 0.1 to 50 parts by weight, and more preferably about 0.3 to 30 parts by weight, to 100 parts by weight of the compound having methyl group or methylene group. A rearrangement reaction using additives may be conducted at temperatures of, for example, about 40°C to 120°C, and preferably about 50°C to 100°C for a period of, for example, about 5 to 180 minutes, and preferably about 10 to 120 minutes. A rearrangement reaction by heating may be conducted at heating temperatures of, for example, about 120°C to 250°C, and preferably about 150°C to 200°C for reaction periods of, for example, about 0.5 to 120 minutes, and preferably about 2 to 90 minutes.

An amide or lactam can be prepared from a corresponding compound having methyl group or methylene group in one step by adding a cyclic compound which contains the structure represented by Formula (1) as a ring constituent and a fluorinated alcohol to the oxime producing reaction system including the compound having methyl group or methylene group, a nitrous ester or nitrite, and a nitrogen-containing cyclic compound which contains the skeleton represented by Formula (8) as a ring constituent. In another embodiment, a reaction between a compound having methyl group or methylene group and a nitrous ester or nitrite is conducted in the presence of a nitrogen-containing cyclic compound which contains the skeleton represented by Formula (8) as a ring constituent and a cyclic compound which contains the structure represented by Formula (1) as a ring constituent to give an oxime compound, and then, a fluorinated alcohol is added after the formation of the oxime compound to carry out the rearrangement reaction of the oxime compound. In still another embodiment, a reaction between a compound having methyl group or methylene group and a nitrous ester or nitrite is conducted in the presence of a nitrogen-containing cyclic compound which contains the skeleton represented by Formula (8) as a ring constituent and a fluorinated alcohol to give an oxime compound, and then, a cyclic compound which contains the structure represented by Formula (1) as a ring constituent is added after the formation of the oxime compound to carry out the rearrangement reaction of the oxime compound. In yet another embodiment, a reaction between a compound having methyl group or methylene group and a nitrous ester or nitrite is conducted in the presence of a nitrogen-containing cyclic compound which contains the skeleton represented by Formula (8) as a ring constituent to give an oxime compound, and then, a cyclic compound which contains the structure represented by Formula (1) as a ring constituent and a fluorinated alcohol are added to carry out the rearrangement reaction of the oxime compound. In these techniques, one or more procedures such as evaporation of solvents, concentration, and solvent exchange may be conducted at suitable periods. The formation of an oxime compound may be conducted stepwise as described above.

The processes according to the present invention simply produce amides or lactams in high yields without causing large amounts of by-products. They also simply produce amides or lactams with high degrees of purification, because catalysts and other components used herein are easily separable from the produced amides or lactams. They further simply and efficiently produce amides or lactams, because the step of yielding an oxime from a starting material such as an aliphatic or aromatic hydrocarbon and the step of yielding an amide or lactam from the oxime compound can be conducted in one step or in one pot. Typically, ε-caprolactam and ω-laurolactam can be efficiently produced from cyclohexane and cyclododecane, respectively.

The produced amides or lactams are usable as raw materials typically for pharmaceutical drugs, agricultural chemicals, dyestuffs, solvents, and explosives and as starting materials for polyamides (nylons) and are industrially very important.

### EXAMPLES

The present invention will be illustrated in further detail with reference to several examples below, which, however, are by no means intended to limit the scope of the present invention.

### (EXAMPLE 1)

Cyclododecanone oxime (10 mmol), cyanuric chloride (0.5 percent by mole to cyclododecanone oxime), and hexafluoroisopropanol (5 ml) were placed in a reactor, followed by stirring under reflux conditions for 2 hours. A gas chromatographic analysis was conducted after the reaction to find that laurolactam was formed in a yield of 99%.

### (EXAMPLE 2)

Cyclohexanone oxime (10 mmol), cyanuric chloride (10 percent by mole to cyclohexanone oxime), and hexafluoroisopropanol (5 ml) were placed in a reactor, followed by stirring under reflux conditions for 2 hours. A gas chromatographic analysis was conducted after the reaction to find that caprolactam and a compound represented by following Formula (11) were formed in yields of 58% and 27%, respectively.

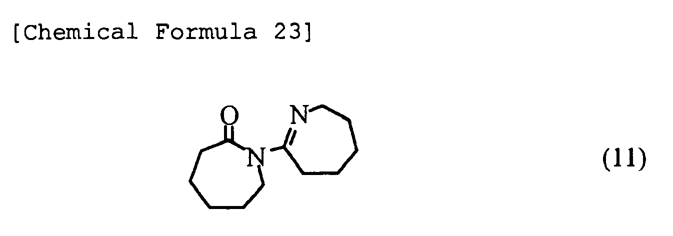

### (EXAMPLE 3)

Cyclohexane (4 ml), t-butyl nitrite (4 mmol), N-hydroxyphthalimide (0.2 mmol), and acetic acid (0.5 ml) were placed in a flask, followed by stirring at 75°C in an argon atmosphere (1 atm = 0.101 MPa) for 2 hours. After the reaction, cyclohexane and acetic acid were evaporated and removed, and the residue was combined with triethylamine (0.5 g) and ethyl acetate (1 ml), followed by stirring at 70°C for 1 hour. Thereafter ethyl acetate and triethylamine were evaporated and removed, the residue was combined with hexafluoroisopropanol (2 ml) and cyanuric chloride (0.2 mmol), followed by stirring under reflux conditions for 2 hours. A gas chromatographic analysis was conducted after the reaction to find that 1.3 mmol of caprolactam was formed.

### (EXAMPLE 4)

Cyclohexane (4 ml), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, cyclohexane and acetic acid were evaporated and removed, and the residue was combined with triethylamine (0.5 g) and ethyl acetate (1 ml), followed by stirring at 70°C for 1 hour. Thereafter ethyl acetate and triethylamine were evaporated and removed, the residue was combined with hexafluoroisopropanol (2 ml) and cyanuric chloride (0.2 mmol), followed by stirring under reflux conditions for 2 hours. A gas chromatographic analysis was conducted after the reaction to find that 2.1 mmol of caprolactam was formed.

### (EXAMPLE 5)

Cyclohexane (4 ml), t-butyl nitrite (4 mmol), N-hydroxyphthalimide (0.2 mmol), and acetic acid (0.5 ml) were placed in a flask, followed by stirring at 75°C under reduced pressure [600 mmHg (79.8 kPa)] for 2 hours. After the reaction, cyclohexane and acetic acid were evaporated and removed, and the residue was combined with triethylamine (0.5 g) and ethyl acetate (1 ml), followed by stirring at 70°C for 1 hour. Thereafter ethyl acetate and triethylamine were evaporated and removed, the residue was combined with hexafluoroisopropanol (2 ml) and cyanuric chloride (0.2 mmol), followed by stirring under reflux conditions for 2 hours. A gas chromatographic analysis was conducted after the reaction to find that 2.0 mmol of caprolactam was formed.

### (EXAMPLE 6)

Cyclohexane (4 ml), N-hydroxyphthalimide (0.2 mmol), and acetic acid (0.5 ml) were placed in a flask, and t-butyl nitrite (4 mmol) was added dropwise thereto at 75°C under reduced pressure [600 mmHg (79.8 kPa)] over 2 hours for a reaction. After the reaction, cyclohexane and acetic acid were evaporated and removed, and the residue was combined with triethylamine (0.5 g) and ethyl acetate (1 ml), followed by stirring at 70°C for 1 hour. Thereafter ethyl acetate and triethylamine were evaporated and removed, and the residue was combined with hexafluoroisopropanol (2 ml) and cyanuric chloride (0.2 mmol), followed by stirring under reflux conditions for 2 hours. A gas chromatographic analysis was conducted after the reaction to find that 2.1 mmol of caprolactam was formed.

### (EXAMPLE 7)

Cyclohexane (4 ml), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, cyclohexane and acetic acid were evaporated and removed, and the residue was combined with triethylamine (0.5 g), followed by stirring at 70°C for 1 hour. Thereafter triethylamine was evaporated and removed, and the residue was combined with hexafluoroisopropanol (2 ml) and cyanuric chloride (0.2 mmol), followed by stirring under reflux conditions for 2 hours. A gas chromatographic analysis was conducted after the reaction to find that 1.9 mmol of caprolactam was formed.

### (EXAMPLE 8)

Cyclohexane (4 ml), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, and sulfuric acid (0.05 g) was added, followed by stirring at 70°C for 1 hour. Thereafter cyclohexane and acetic acid were evaporated and removed, and the residue was combined with hexafluoroisopropanol (2 ml) and cyanuric chloride (0.2 mmol), followed by stirring under reflux conditions for 2 hours. A gas chromatographic analysis was conducted after the reaction to find that 1.8 mmol of caprolactam was formed.

### (EXAMPLE 9)

cyclohexane (4 ml), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, and an acid-type ion-exchange resin (AMBERLYST 15DRY; 0.05 g) was added, followed by stirring at 70°C for 1 hour. After filtration, cyclohexane and acetic acid were evaporated and removed, and the residue was combined with hexafluoroisopropanol (2 ml) and cyanuric chloride (0.2 mmol), followed by stirring under reflux conditions for 2 hours. A gas chromatographic analysis was conducted after the reaction to find that 1.9 mmol of caprolactam was formed.

### (EXAMPLE 10)

Cyclohexane (4 ml), t-butyl nitrite (4 mmol), N-hydroxyphthalimide (0.2 mmol), and acetic acid (0.5 ml) were placed in a pressure-tight glass reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, and the mixture was heated to 180°C, followed by heating at this temperature for 1 hour. Thereafter cyclohexane and acetic acid were evaporated and removed, and the residue was combined with hexafluoroisopropanol (2 ml) and cyanuric chloride (0.2 mmol), followed by stirring under reflux conditions for 2 hours. A gas chromatographic analysis was conducted after the reaction to find that 1.5 mmol of caprolactam was formed.

### (EXAMPLE 11)

Cyclohexane (4 ml), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. Thereafter the reduced pressure was released to normal pressure, hexafluoroisopropanol (2 ml) and cyanuric chloride (0.2 mmol) were added, followed by stirring under reflux conditions for 2 hours. A gas chromatographic analysis was conducted after the reaction to find that 1.8 mmol of caprolactam was formed.

### (EXAMPLE 12)

Cyclododecane (3 g), t-butyl nitrite (4 mmol), N-hydroxyphthalimide (0.2 mmol), and acetic acid (0.5 ml) were placed in a reactor, and the resulting solution was frozen. The reactor was evacuated, adjusted in pressure to 50 mmHg (6.65 kPa) with argon gas, and sealed, followed by stirring at 75°C for 2 hours. After the reaction, the reduced pressure was released to normal pressure, cyclohexane and acetic acid were evaporated and removed, and the residue was combined with triethylamine (0.5 g) and ethyl acetate (1 ml), followed by stirring at 70°C for 1 hour. Thereafter ethyl acetate and triethylamine were evaporated and removed, and the residue was combined with hexafluoroisopropanol (2 ml) and cyanuric chloride (0.02 mmol), followed by stirring under reflux conditions for 2 hours. A gas chromatographic analysis was conducted after the reaction to find that 2.9 mmol of laurolactam was formed.

### (REFERENTIAL EXAMPLE 1)

### [Synthesis of 2,4,6-tris(hexafluoroisopropyloxy)-1,3,5-triazine (2,4,6-tris(2,2,2-trifluoro-1-trifluoromethylethoxy)-1,3,5-triazine)]

Cyanuric chloride was added with 5 times, by equivalent weight to cyanuric chloride, of hexafluoroisopropanol and 3.5 times, by equivalent weight to cyanuric chloride, of triethylamine, and the mixture was stirred in THF at room temperature for 5 hours. After the reaction, the solvent was evaporated and removed, the residue was purified by column chromatography and thereby yielded 2,4,6-tris(hexafluoroisopropyloxy)-1,3,5-triazine.

### (EXAMPLE 13)

Cyclododecanone oxime (10 mmol), 2,4,6-tris(hexafluoroisopropyloxy)-1,3,5-triazine (2,4,6-tris(2,2,2-trifluoro-1-trifluoromethylethoxy)-1,3,5-triazine) (0.5 percent by mole), and hexafluoroisopropanol (2 ml) were placed in a reactor, followed by stirring under reflux conditions for 2 hours. A gas chromatographic analysis was conducted after the reaction to find that laurolactam was formed in a yield of 99%.

### (EXAMPLE 14)

Cyclododecanone oxime (10 mmol), 2,4,6-tris(cyclododecylideneaminooxy)-1,3,5-triazine (O-4,6-bis(cyclododecylideneaminooxy)-1,3,5-triazin-2-ylcyclododecanone oxime) (0.5 percent by mole), and hexafluoroisopropanol (2 ml) were placed in a reactor, followed by stirring under reflux conditions for 2 hours. A gas chromatographic analysis was conducted after the reaction to find that laurolactam was formed in a yield of 99%.

### Industrial Applicability

According to the present invention, rearrangement reactions of oximes can be carried out without causing large amounts of by-products such as ammonium sulfate, to thereby solve problems on removal and disposal of by-products formed in known processes for the production of amides or lactams, and amides or lactams can be simply produced in high yields.

## Claims

1. A process for the production of amides or lactams, comprising the step of carrying out a rearrangement of an oxime compound in the presence of a fluorinated alcohol and a cyclic compound containing a structure represented by following Formula (1) as a ring constituent to yield a corresponding amide or lactam: 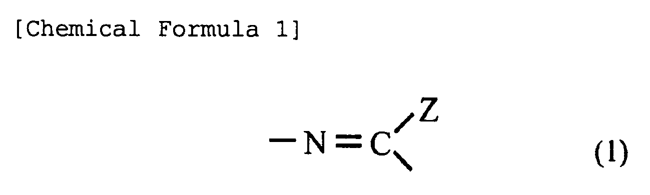 wherein Z represents a halogen atom or an -OR group, where R represents an organic group.

2. The process for the production of amides or lactams of claim 1, wherein Z is chlorine atom.

3. The process for the production of amides or lactams of claim 1, wherein the fluorinated alcohol is a fluorine-containing branched-chain aliphatic alcohol represented by following Formula (3): 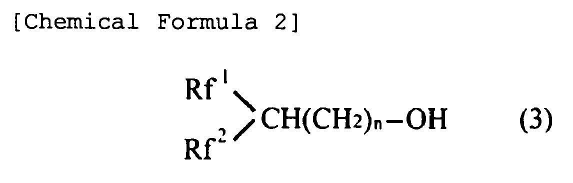 wherein Rf¹ and Rf² may be the same as or different from each other and each represent a perfluoroalkyl group having one to eight carbon atoms; and "n" denotes an integer of 0 to 8.

4. The process for the production of amides or lactams of claim 3, wherein R in Formula (1) is a fluorine-containing branched-chain aliphatic group represented by following Formula (3a): 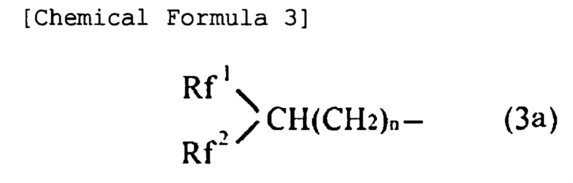 wherein Rf¹ and Rf² may be the same as or different from each other and each represent a perfluoroalkyl group having one to eight carbon atoms; and "n" denotes an integer of 0 to 8.

5. The process for the production of amides or lactams of claim 1, wherein the oxime compound is represented by following Formula (4) or (5): 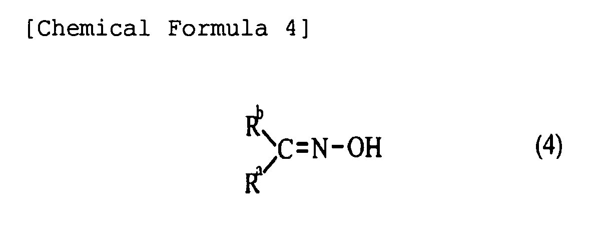 wherein R^{a} and R^{b} each independently represent an organic group, where one of R^{a} and R^{b} may be hydrogen atom, 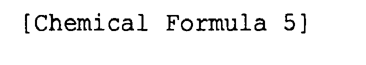 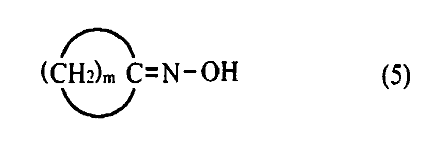 wherein "m" denotes an integer of 2 or more.

6. The process for the production of amides or lactams of claim 5, wherein R in Formula (1) is a group corresponding to the oxime compound and represented by following Formula (4a) or (5a): 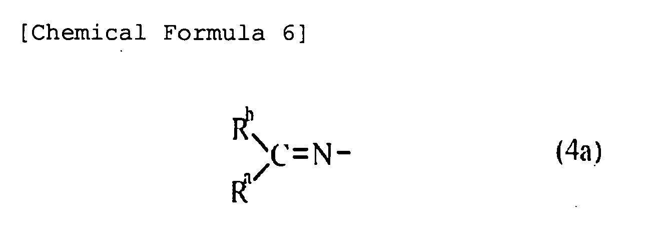 wherein R^{a} and R^{b} each independently represent an organic group, where one of R^{a} and R^{b} may be hydrogen atom, 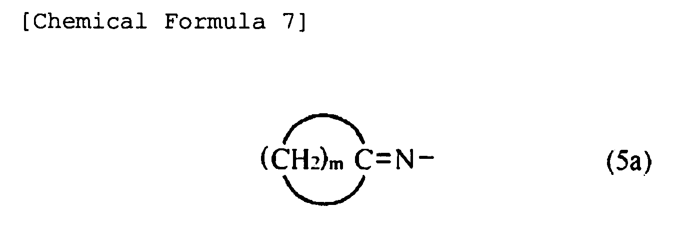 wherein "m" denotes an integer of 2 or more.
